# EUROPÄISCHE PATENTSCHRIFT

(11) **EP 0 513 650 B1**
(45) Veröffentlichungstag und Bekanntmachung des Hinweises auf die Patenterteilung: **23.08.1995**
(21) Anmeldenummer: 92107656.8
(22) Anmeldetag: 06.05.1992
(51) Int. Cl.: A61M 16/04, A61M 25/00, A61L 29/00

(54) **Trachealtubus mit permeationsstabiler Blockungsmanschette**
Tracheal tube with blocking sleeve with stabilized permeation
Tube trachéal avec manchon de blocage ayant une perméation stabilisée

(30) Priorität: 11.05.1991 DE 4115497
(43) Veröffentlichungstag der Anmeldung: 19.11.1992
(73) Patentinhaber: WILLY RÜSCH AG, D-71385 Kernen (DE)
(72) Erfinder: Kepp, Werner, W-7050 Waiblingen (DE); Schmitt, Klaus, W-7064 Remshalden (DE)
(74) Vertreter: KOHLER SCHMID + PARTNER

(56) Entgegenhaltungen:
- EP-A- 0 204 500
- WO-A-84/01294
- GB-A- 2 008 140
- US-A- 3 503 399
- US-A- 4 335 723

## Beschreibung

Die Erfindung geht aus von einem Trachealtubus mit einer an einem Schaft angeordneten permeationsstabilen Blockungsmanschette, für die ein Material in ausreichender Schichtdicke verwendet wird und wobei die Blockungsmanschette einschichtig aufgebaut ist.

Ein derartiger Trachealtubus ist durch die US 4,335,723 bekanntgeworden.

Die US 4, 335,723 offenbart einen Trachealtubus mit einem Ballon, der im wesentlichen aus einem thermoplastischen Elastomer besteht. Dabei enthält die Wand des Ballons ein Blockpolymer in der Form eines Endblocks eines Styrene-Polymers und einen elastomerischen Block aus offenen, gesättigten Hydrokarbonpolymerketten. Der elastomerische Block kann dabei aus hydriertem Polybutadien oder einem Polyolefingummi wie ethylene-butylene Copolymeren hergestellt sein.

Aus der US 3,503,399 ist ein Trachealtubus bekannt, der einen aufblasbaren Ballon aufweist, der beispielsweise aus natürlichem Gummi hergestellt ist.

Derartige Trachealtuben werden bevorzugt bei Intubationsnarkosen eingesetzt. Nach der Intubation wird die Blockungsmanschette in situ mit Luft gefüllt. Die Blockungsmanschette verschließt dann den Zwischenraum zwischen Trachea und Beatmungsschlauch, so daß das Inspirationsgemisch nicht mehr entweichen kann und in die Lungen strömt. Untersuchungen haben ergeben, daß es während Intubationsnarkosen mit bestimmten Narkosegasen zu einem starken Anstieg des Blockungsmanschettendruckes kommen kann. Die Ursache ist, daß während einer Intubationsnarkose das Narkosegas in die Blockungsmanschette diffundiert. Daraus ergibt sich eine Volumenänderung des Gases in der Blockungsmanschette und sie drückt mit erhöhtem Druck auf die Trachealschleimhaut. Der Druckanstieg in der Blockungsmanschette kann einerseits zu Trachealwandschäden führen und andererseits kann sich die Blockungsmanschette bei zu hohen Drücken zusätzlich plastisch verformen. Erfolgt die plastische Verformung auf der der Tubusspitze zugewandten Seite der Blockungsmanschette, so kann dies sogar zu einer partiellen oder totalen Lumenverlegung des Tubusschaftes im Bereich der Tubusspitze führen.

Die Volumen- und die damit verbundenen Druckänderungen in der Blockungsmanschette hängen unter anderem vom Konzentrationsgefälle des Narkosegases zur Blockungsmanschette hin, von der Einwirkzeit des Narkosegases auf die Blockungsmanschette und dem Grad der Gasdurchlässigkeit der Blockungsmanschette ab.

Der in der DE-39 21 524 A1 offenbarte Trachealtubus weist eine mehrschichtig aufgebaute Niederdruckblockungsmanschette mit großer Oberfläche auf, die relativ dünnwandig ist. Das Manschettenvolumen ist vorgegeben und die Blockungsmanschette kann sich in situ ohne wesentliche Druckerhöhung entfalten. Die Blockungsmanschette ist narkosegasundurchlässig, weil sie mindestens eine Materialschicht aufweist, beispielsweise eine Metallfolie, die narkosegasundurchlässig ist. Die bei der bekannten Blockungsmanschette verwendeten Materialien müssen sorgfältig aufeinander abgestimmt sein, so daß sie sowohl im drucklosen, wie auch im druckbeaufschlagten Zustand formschlüssig aneinander liegen, damit unerwünschte Falten auszuschließen sind. Ferner muß in den Übergangsbereichen zum Schaft ein sehr sorgfältiger Materialverbund erstellt werden, damit hier nicht bevorzugt Diffusionsbereiche entstehen können.

Der Erfindung liegt daher die Aufgabe zugrunde, einen Trachealtubus der eingangs genannten Art dahingehend weiterzubilden, daß er eine im Aufbau einfache Blockungsmanschette aufweist, bei der in den üblichen Anwendungszeiträumen kein oder nur eine vernachlässigbar kleine Menge Narkosegas von der Trachea in die Blockungsmanschette diffundieren kann.

Die Aufgabe wird erfindungsgemäß dadurch gelöst, daß das Material die Struktur eines Butadienacrylnitrilmischpolymerisates aufweist, das einen Acrylnitrilgehalt zwischen 25% und 35% aufweist.

Der Materialtrukturaufbau der erfindungsgemäßen Blockungsmanschette ist derart, daß beispielsweise N₂O-Moleküle nicht in einem wesentlichen Umfang hindurchtreten können. Mit dem Einsatz einer derartigen Blockungsmanschette können druckinduzierte Schleimhautläsionen ausgeschlossen werden. Eine dauerhafte und schleimhautschonende Abdichtung zwischen Tubusschaft und Trachea wird erreicht.

Geeignet vernetzte Elastomere aus Nitrit- oder Chlorkautschuk z.B. wie Butadien, sind neben einer guten Permeabilitätsstabilität auch besonders thermostabil. Deshalb sind Tuben mit einer erfindungsgemäßen Blockungsmanschette besonders gut resterilisierbar. Gemäß der Erfindung werden für die Herstellung der Blockungsmannschette Butadienacrylnitrilmischpolymerisate verwendet, die gegenüber organischen Lösungsmitteln weitgehend resistent sind. Ein Aufquellen oder ein Anlösen des Mischpolymerisats durch den Kontakt mit organischen Lösungsmitteln wird verhindert.

Trachealtuben mit der erfindungsgemäßen Blockungsmanschette können kostengünstiger hergestellt werden, weil ein zusätzlicher Schichtaufbau bzw. zusätzliche Komponenten entfallen können.

Der einschichtige Aufbau der Blockungsmanschette hat den Vorteil, daß sie in einer sehr dünnen Wandstärke gefertigt werden kann und eine in der Anwendung nachteilige Faltenbildung vermieden wird. Die Materialstärke liegt dabei zwischen 0,1 und 0,15 mm.

Nitril- oder Chlorkautschuk ist wie bekannt sehr gut gegen organische Lösungsmittel beständig. Weiterhin ist bekannt, aus Anwendungsbereichen im Automobilbau, daß diese Kunstkautschuke ölunempfindlich sind. Narkosegase sind als halogenierte Kohlenwasserstoffe sehr stark lipophil. Nitrilkautschuk ist auch, wie Versuche ergaben, gegenüber halogenierten Kohlenwasserstoffen ausreichend beständig und auch insoweit thermostabil, daß Trachealtuben mit der erfindungsgemäßen Blockungsmanschette vielfach resterilisiert werden können. Nitrilkautschuk läßt sich auch in Verbindung mit Gummi bzw. Latex gut verarbeiten, so daß der Schaft eines Trachealtubus aus Gummi bzw. Latex und/oder Chlorkautschuk bestehen kann, während die Blockungsmanschette aus Nitrilkautschuk gefertigt ist. Selbstverständlich können aber auch andere Kunstkautschuke als Schaftmaterial verwendet werden. Die Diffusionskapazität von Nitrilkautschuk für Narkosegase ist außerordentlich gering.

Ein Mischungsverhältnis für das Material der Blockungsmanschette von 30 % Acrylnitril und 70 % Butadien hat sich besonders bewährt.

Der erfindungsgemäße Trachealtubus entspricht damit allen erweiterten Anforderungen in der Anästhesie. Er ist als highvolume-/lowpressure-cuff schleimhautschonend, er bietet hinreichende Sicherheit gegen eine unzulässig starke Narkosegasdiffusion und kann sowohl als Einmaltubus wie auch als mehrfach verwendbarer Tubus ausgebildet sein.

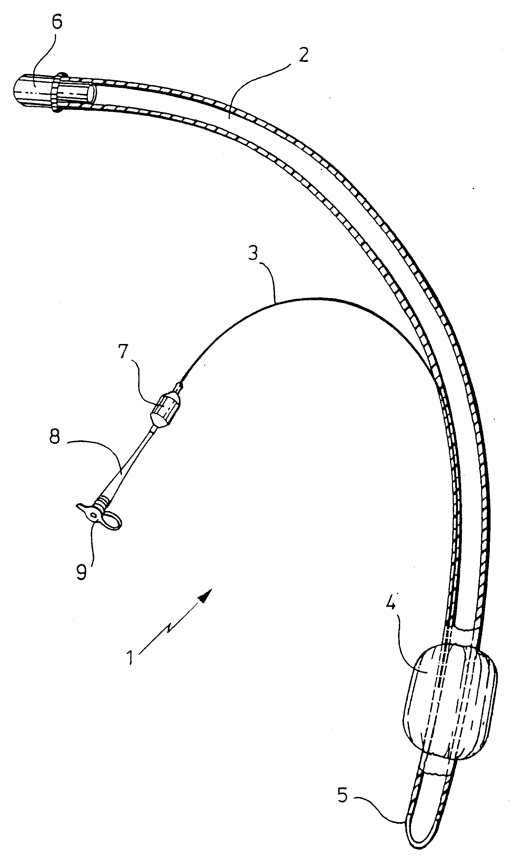

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand einer Figur erläutert. Der in der Figur dargestellte Gegenstand zeigt teilweise stark schematisiert die erfindungsgemäße Blockungsmanschette. Der Gegenstand ist nicht maßstäblich zu verstehen.

Die Figur zeigt einen Trachealtubus 1, der an einem Tubusschaft 2 einen Zuführungsschlauch 3 aufweist. Der Zuführungsschlauch 3 ist bevorzugt in der Wandung des Tubusschaftes 2 geführt und steht mit einer Blockungsmanschette 4 in Verbindung, die am Tubusschaft 2 befestigt ist. Die Blockungsmanschette 4 ist kurz vor einer Spitze 5 des Tubusschaftes 2 angeordnet. Die Blockungsmanschette 4 ist einschichtig aufgebaut und im Ausführungsbeispiel aus Kunstkautschuk gefertigt. Bei der in der Figur dargestellten Blockungsmanschette handelt es sich um einen highvolume-/lowpressure-cuff.

Am maschinenseitigen Ende des Tubusschaftes 2 ist ein genormter Konnektor 6 vorgesehen, über den die Schläuche einer Beatmungsmaschine mit dem Trachealtubus 1 gasdicht verbunden werden können (genormte Schnittstelle). Der Zuführungsschlauch 3 weist im Bereich seines freien Endes einen Kontrollballon 7 auf, an den sich ein Spritzenansatz 8 anschließt. Der Spritzenansatz 8 ist mittels eines Verschlusses 9 verschließbar. Über den Spritzenansatz 8 kann die Blockungsmanschette 4 in einer in der Figur nicht dargestellten Art und Weise entweder mit Luft befüllt oder entlüftet werden.

Die Blockungsmanschette 4 ist aus einem narkosegasundurchlässigen Material gefertigt, das den Strukturaufbau eines Butadienacrylnitrilmischpolymerisats oder eines Chloroprenepolymerisates aufweist. Der Tubusschaft kann aus PVC, Polyurethan bzw. Gummi, Kunstkautschuk oder Latex gefertigt sein.

Die Materialstärke der aus einem vernetzten Elastomer gefertigten Blockungsmanschette beträgt 0,1 bis 0,15 mm und das Mischungsverhältnis bei der Verwendung eines Butadienacrylnitrilmischpolymerisats kann zwischen 25 % und 35 % Acrylnitril und 65 % bis 75 % Butadien betragen.

## Patentansprüche

1. Trachealtubus mit einer an einem Schaft (2) angeordneten permeationsstabilen Blockungsmanschette (4), für die ein Material in ausreichender Schichtdicke verwendet wird, und wobei die Blockungsmanschette (4) einschichtig aufgebaut ist,
dadurch gekennzeichnet, daß das Material die Struktur eines Butadienacrylnitrilmischpolymerisats aufweist, das einen Acrylnitrilgehalt zwischen 25 % und 35 % aufweist.

2. Trachealtubus nach Anspruch 1,
dadurch gekennzeichnet, daß die Schichtdicke der Blockungsmanschette (4) zwischen 0,1 mm und 0,15 mm liegt.

3. Trachealtubus nach Anspruch 1,
dadurch gekennzeichnet, daß das elastomere Butadienacrylnitrilmischpolymerisat eine große Beständigkeit gegenüber organischen Lösungsmitteln aufweist.

## Claims

1. Tracheal tube with a permeation-stable blocking cuff (4) arranged at a shaft (2), a material of sufficient layer thickness being used for the blocking cuff (4) and the blocking cuff (4) having a single-layer construction, characterized in that the material has the structure of a butadiene acrylnitrile mixed polymer with an acrylnitrile content between 25% and 35%.

2. Tracheal tube according to claim 1, characterized in that the layer thickness of the blocking cuff (4) is between 0.1 mm and 0.15 mm.

3. Tracheal tube according to claim 1, characterized in that the elastomeric butadiene acrylnitrile mixed polymer is highly resistant to organic solvents.

## Revendications

1. Tube trachéen comportant un manchon de blocage (4) résistant à la perméation, disposé sur une tige (2), pour lequel est utilisé un matériau d'épaisseur de couche suffisante et le manchon du blocage (4) ayant une structure monocouche, caractérisé en ce que le matériau a la structure d'un copolymère butadiène-acrylonitrile présentant une teneur en acrylonitrile comprise entre 25 % et 35%.

2. Tube trachéen selon la revendication 1, caractérisé en ce que l'épaisseur de couche du manchon de blocage (4) est comprise entre 0,1 mm et 0,15 mm.

3. Tube trachéen selon la revendication 1, caractérisé en ce que le copolymère butadiène-acrylonitrile élastomère présente une grande résistance aux solvants organiques.
